Europäisches Patentamt

European Patent Office

Office européen des brevets

(11) Publication number: **0 230 649**
**A1**

# EUROPEAN PATENT APPLICATION

(21) Application number: 86117965.3

(22) Date of filing: 23.12.86

(51) Int. Cl.4: **C12N 11/10 , C12N 11/02 ,
C12N 9/88**

(30) Priority: 02.01.86 US 815627

(43) Date of publication of application:
05.08.87 Bulletin 87/32

(84) Designated Contracting States:
DE GB

(71) Applicant: **MILES LABORATORIES, INC.
1127 Myrtle Street
Elkhart Indiana 46514(US)**

(72) Inventor: **Borchert, Peter J.
28993 Ella Drive
Elkhart, IN 46515(US)**
Inventor: **Lantero, Oreste J.
59731 Ridgewood Drive
Goshen, IN 46526(US)**
Inventor: **Neff, Jerry L.
28440 County Road 52 R.R. No. 4
Nappanee, IN 46550(US)**

(74) Representative: **Jesse, Ralf-Rüdiger, Dr. et al
Bayer AG Konzernverwaltung RP
Patentabteilung
D-5090 Leverkusen 1 Bayerwerk(DE)**

(54) Immobilization of phenylaline ammonia-lyase.

(57) Disclosed is a process whereby cells of phenylalanine ammonia-lyase producing *Sporobolomyces roseus* are immobilized. The process involves treating the *S. roseus* with a polyamine compound having pendant amino groups, a multifunctional amine reactive material, and chitosan. The immobilized *S. roseus* is useful in the bioconversion of trans cinnamic acid to L-phenylalanine, which product can be converted to its lower alkyl ester which is useful in the manufacture of the sweetener aspartame.

EP 0 230 649 A1

## IMMOBILIZATION OF PHENYLALINE AMMONIA-LYASE

### BACKGROUND OF THE INVENTION

The use of enzymes derived from microbial cells to effect specific chemical transformations is well known. In carrying out such transformations, the cell-free enzyme preparation, ruptured cells or whole cells can be used as the source of biocatalyst. The free enzymes or cells can be efficiently used in batch-type processes but such processes do not lend themselves to continuous industrial scale operations as the enzymes or cells are difficult to remove for reuse from solutions or suspensions in which they are utilized. For example, Japanese Kokai 81 26197 (1981) and Japanese Kokai 78 96388 (1978), both of which are assigned to Tanabe Seiyaku Co., Ltd., disclose a batch process in which cells of *Sporobolomyces roseus* containing PAL enzyme (phenylalanine ammonium-Lyase, sometimes referred to as phenylalanine ammonia-lyase) are dispersed in a substrate solution of cinnamic acid and $NH_4+$ or $NH_3$ to effect the enzymatic production of L-phenylalanine (L-phe). This product can be converted to its lower alkyl ester which is useful in the production of the diet sweetener, aspartame (alpha-L-aspartyl-L-phenylalanine methyl ester). See Klausner, "Building for Success in Phenylalanine", Biotechnology, Vol. 3, No. 4, pages 301-307 (April, 1985), which compares the different aspartame production methods used by Ajinomoto, Searle, Bio Technica, Genex, Degussa, Synthetech, Purification Engineering, and W.R. Grace. For more details on aspartame production from L-phe, see for instance U.S. Patent 4,394,308, European Patent 93128, U.S. Patent 4,111,925, European Patent 74095 and British Patent 2092161, the disclosures of which are herein incorporated by reference. Batch processing led to an interest on the part of the present inventors to investigate the preparation of various forms of immobilized biocatalysts, which are better suited for industrial scale operations. This immobilization permits reuse whereas batch processing might result in the enzyme otherwise undergoing deactivation or result in the loss of the cells in the reaction medium used. These immobilized enzyme systems may be employed in various reactor systems, such as in packed columns and stirred tank reactors, depending on the nature of the substrate which is being biocatalytically reacted.

Several general methods as well as many modifications thereof have been described by which immobilization can be effected. For example, materials useful for immobilization of enzymes or cells containing enzymes are disclosed in U.K. Patent No. 1,444,539. Preferably, the enzymes or cells are treated with a water miscible solvent, such as acetone, dried and then treated with polyethylenimine and glutaraldehyde to make shaped bodies of a water insoluble structure.

The Genex enzymatic production of phenylalanine (for use in making aspartame) which is mentioned in the Klausner article discussed above, is described in detail in Example V of U.S. Patent 4,434,228. This Example shows immobilization of *Rhodotorula rubra* containing PAL enzyme using an immobilization system consisting of polyethylenimine, succinic acid, and a carbodiimide HC1. Example V further describes how the immobilized *R. rubra* containing PAL enyzme can then be treated with cinnamic acid (as depicted in Figure II on page 306 of the Klausner article) to convert it to L-phe. After 39 hours, the conversion resulted in only 0.5 grams/liter of L-phe.

### STATEMENT OF THE INVENTION

Disclosed is a method for the immobilization of phenylalanine ammonia-lyase producing *Sporobolomyces roseus* which method comprises forming a reaction product by the steps of: a) at a temperature less than about 20°C, introducing a solution of a polyamine having pendant amino groups to an aqueous medium containing a mass of cells of *S. roseus*; b) adding a multifunctional amine reactive material and a solution of chitosan to the aqueous medium to form a cross-linked reaction product; and c) removing the cross-linked reaction product from the aqueous medium to provide an immobilized cell mass end product capable of catalyzing the biotransformation of trans cinnamic acid to L-phenylalanine.

### DESCRIPTION OF THE INVENTION

The method of the present invention is useful for the immobilization of Sporobolomyces roseus containing the intracellular enzyme phenylalanine ammonia-lyase.

The cells to be immobilized are typically grown in an aqueous nutrient medium and concentrated either by filtration or centrifugation to provide an aqueous medium containing about 10% (by weight) dry matter. The enzyme containing cells are immobilized by the addition of a polyamine, chitosan, and a multifunctional amine reactive material such as glutaraldehyde to the aqueous medium.

Specific examples of polyamines suitable for use in the present invention include polyethylenediamine, polyethylenimine, polydiethylenetriamine, polytriethylenetetramine, polypentaethylenehexamine, or polyhexamethylenediamine. Also, Betz 1180® may be used. This is a water soluble copolymer of epihalohydrin and a polyamine and is marketed by Betz Laboratories, Inc., Trevose, Pennsylvania. Other suitable polyamines are polymethylenedicyclohexylamine, polymethylenedianiline, polytetraethylenepentamine and polyphenylenediamine. The primary amino groups of the polyamine should be at least about 10 weight percent of the total amino group content of the polyamine with the preferred amount being about 25 weight percent. The polyamine is preferably placed in solution, preferably at a concentration from about 1 to 10 weight percent before its addition to the enzyme containing medium, so that adequate mixing of the polyamine into the cell mixture can be obtained.. Those polyamines which are water soluble are applied from their aqueous solutions whereas non-water soluble polyamines are applied from organic solvents. Suitable solvents include, but are not limited to, methyl alcohol, ethyl alcohol, propyl alcohol, isopropyl alcohol, t-butyl alcohol, acetone, methyl ether, ethyl ether, propyl ether, isopropyl ether, toluene, benzene, zylene, hexane, cyclopentane and cyclohexane. Typically, the amount of polyamine added will be from about 2 to 22 weight percent of the immobilized cell mass preparation.

While the molecular weight of the polyamine is not deemed critical, those with a molecular weight range from about 500 to 100,000 are preferred. The more preferred molecular weight range is from about 40,000 to 80,000 Daltons. Immobilizations carried out using low molecular weight (below about 40,000 Daltons) result in cell aggregation; however, the aggregated cell mass can not be readily recovered by filtration, as the physical properties of the cell aggregate appear to be somewhat different, such that the cell aggregate tends to bind off the filter paper. Since the aggregated cell mass is actually in an insoluble form, the cell aggregate could be recovered by centrifugation, another common method of recovery. The packed-cell aggregate could then be extruded as further discussed below and shaped in the usual manner to obtain the desired immobilized enzyme particle matrix.

Next, a cross-linked reaction product is formed by the addition of a multifunctional amine reactive material such as glutaraldehyde and a solution of chitosan to the medium. These reactants can be added in any order or simultaneously.

The term chitosan as used herein is intended to mean a polyamino polysaccharide obtained by N-deacetylation of chitin with strong alkali and heat. Chitin is a polysaccharide wherein the primary repeating unit in the molecule is 2-deoxy-2-(acetylamino)-glucose. In general, about one out of every six units in chitin is not acetylated, whereas in chitosan essentially none of the repeating units is acylated. The degree of acetylation can be controlled by the severity of the deacetylation reaction. Chitin, which is distributed widely in nature such as in marine invertebrates, insects, fungi and yeast, can be readily prepared by removing the impurities from shells of crab, shrimp, lobsters, crayfish and the like which are abundantly available from seafood processing plants. Typically, the amount of chitosan employed will be from about 0.5 to 22 weight percent of the immobilized cell mass preparation with an amount of from about 3.5 to 10 weight percent being preferred.

The chitosan should be placed in aqueous solution before its addition to the enzyme containing medium. It is normally insoluble in water; however, it is soluble in dilute aqueous acid; i.e. formic, acetic, pyruvic, lactic, malic, citric, and inorganic acids with the exception of sufuric acid in which there is formed an insoluble chitosan sulfate complex. To be effective in the present immobilizaton process, chitosan must become an integral portion of the immobilized cell matrix. Therefore, chitosan preferably is placed in dilute aqueous acidic solution before use. A solution containing about 0.2 to 1.0 weight percent chitosan is preferred and is prepared by adding the chitosan to a 0.5% solution of acetic acid. Heating the solution to about 60°C helps dissolve the chitosan. After heating, the chitosan solution is filtered to remove insoluble residue.

Examples of multifunctional amine reactive materials are glutaraldehyde; bis-diazobenzidine-2,2'-disulfonic acid; 4,4'-difluoro-3,3'dinitrodiphenylsulfone; diphenyl-4,4'-dithiocyanate-2,2'-disulfonic acid; 3-methoxydiphenylmethane-4,4'-diisocyanate; toluene-2-isocyanate-4-isothiocyanate; toluene-2,4-diisothiocyanate; diazobenzidine; diazobenzidine-3,3'-dianisidine; N,N'-hexamethylene bisiodoacetamide; hexamethylene diisocyanate; cyanuric chloride; and 1,5-difluoro-2,4-dinitrobenzene. The material is desirably applied from a solution preferably containing from approximately 1 to 100 gram per liter of the multifunctional amine reactive material. Those amine reactive materials which are water soluble are applied from their aqueous solutions whereas non-water soluble materials are applied from organic solvents. Suitable solvents include but are not limited to those mentioned above for the polyamine.

Preferably, the multifunctional amine reactive material employed in this invention is glutaraldehyde. It is preferably employed in an amount from about 4 to 26 weight percent of the immobilized cell mass preparation with an amount of about 7 to 15 weight percent being preferred. It is noted that glutaraldehyde, when in aqueous solution, liberates H$^+$, and thus it may be advantageous during the immobilization to add some base, such as NaOH, to bring the pH up should it drop into the acid range which may tend to denature the PAL enzyme. Thus, regardless of what multifunctional amine reactive material is used, the pH during immobilization should be maintained above about 7.0.

The biocatalyst, polyamine, chitosan and multifunctional amine reactive material combine to form a wet cake of immobilized enzyme preparation which can be removed from the reaction medium by liquid/solid separatory techniques, dried, broken into particles and used for the intended biocatalytic conversion. However, a more desirable physical form of the material can be obtained by extruding, drying, and grinding. Also, the wet cake may be dried and then extruded onto the rotating plate of a spheronizing device which comprises a milled friction plate as rotor situated in a cylinder such that the cylinder provides a stationary wall for the plate while allowing it freedom to rotate.

The aqueous feed substrate solutions, used in the Examples below to test the effectiveness of PAL in converting trans cinnamic acid to L-phenylalanine, were prepared as follows.

#### 1% w/v cinnamic acid substrate solution:

10 grams of trans cinnamic acid were dissolved in 200 ml of concentrated ammonium hydroxide (59.2 g NH$_3$ = 3.482 M) and 500 ml of deionized water. 150 ml of concentrated hydrochloric acid (57 grams HC1 = 1.56 M) were slowly added under external cooling. Finally, a total of 0.246 grams of MgSO$_4$•7H$_2$O were added and the volume brought to 1000 ml with water. The solution was divided into 3 samples and final pH adjustments were made to 9.2 , 9.3, or 10.0, respectively, using dilute hydrochloric acid. The resultant had a cinnamic acid concentration of 1% w/v (0.0675 M), a ratio NH$_3$/cinnamic acid (molar) of 51/1, and an ammonium chloride concentration of 8.34% w/v.

#### 0.5 w/v cinnamic acid substrate solution:

50 ml of the 1% w/v cinnamic acid substrate prepared as described in the paragraph above was diluted to 100 ml by adding 50 ml of deionized water. The resultant had a cinnamic acid concentration of 0.5% w/v (0.0337 M), a ratio NH$_3$/cinnamic acid (molar) of 51/1, and an ammonium chloride concentration of 4.17% w/v.

#### 3% w/v cinnamic acid substrate solution:

The process described above for the 1% w/v substrate was repeated except 30 grams of cinnamic acid and 100 ml of concentrated hydrochloric acid (38 g = 1.04 M) were used. The resultant had a cinnamic acid concentration 3% w/v (0.202 M), a ratio NH$_3$/cinnamic acid (molar) of 17/1, and an ammonium chloride concentration of 5.56% w/v.

The S. roseus employed in the Examples below was prepared from a fermentation as follows.

#### FERMENTATION

A stock culture, S. roseus ATCC #28988, was maintained on 0.39% w/v agar slants containing potato dextrose agar (pH 6.0 adjusted with 10% w/v HC1). 100 ml of a medium was prepared comprising 1% w/v peptone (supplied by Difco Laboratories, Detroit, Michigan), 1% w/v yeast extract (also supplied by Difco), 0.5% w/v sodium chloride, and 0.05% w/v L-Phenylalanine in water in a 250 ml flask and sterilized at 120°C for 10 minutes. 40 ml of medium was placed in a 125 ml Erlenmeyer flask, inoculated with the stock culture, and incubated for 24 hours on a rotary shaker at a temperature ranging from 20-25°C. The resultant was then transferred to a one liter flask containing 500 ml of medium and reincubated in a similar fashion. The resultant was then transferred to a 100 liter fermentor containing 80 liters of medium and aerated with compressed air in the presence of sufficient Dow-Corning antifoam A to suppress foaming. The fermentor was incubated at ambient temperature (25°C ±1) for a total of 32 hours. It is noted that preferably the fermentation temperature should be maintained so that it is not any higher than about 27°C, as PAL enzyme is heat liable. More details on fermentation are given in Example V below.

To monitor enzyme production, at intervals, small samples were withdrawn from the fermentor and the *S. roseus* cells were collected with a centrifuge and decantation of the liquid. The cells had a reddish-brown cast and it is noted that the production of PAL by *S. roseus* is indicated by the appearance of a distinctly reddish cast to the cells. The spontaneous increase of the pH of the broth from 6.0 to 7.6 was monitored. For maximum performance of the cells, it is desirable to have the final pH be approximately 7.2 to 8.0.

If the cells are to be immobilized in the near future, they may be stored at ambient conditions up to about 3 days. Otherwise, they may be stored indefinitely at about 4°C until it is desired to use them.

The method for practicing the present invention is further illustrated by the following examples:

## EXAMPLE I

### CELL ISOLATION

A cell suspension from an entire fermentation broth prepared as described above was centrifuged, followed by decantation of the remaining liquid to separate the centrifugate containing the whole cells from the fermentation broth. Yield: 1800 grams (2.25% wt./vol.) of centrifugate. 560 grams of the centrifugate was stored at 4°C for a total of nine days. After this time, a further separation of free whole cells (about 330 grams) from the remaining liquid took place by gravity. The liquid was decanted and these cells were used in the following immobilization.

### IMMOBILIZATION PROCEDURE

The 330 grams of the free whole cells, as described above, containing phenylalanine ammonia-lyase, were mixed under agitation with 800 ml of deionized water and the suspension cooled to a temperature between 15-18°C. This temperature was maintained during the immobilization. To this suspension was added with stirring 83 ml of a 5% w/v aqueous solution of polyethylenimine (average MW 60,000) over a period of 3-5 minutes. After continued agitation for 20 minutes, 235 ml of a 0.5% w/v dilute aqueous acetic acid solution of chitosan was added within a minute and stirring continued for 15 minutes. Next, 15 ml of a 10% w/v aqueous solution of glutaraldehyde was added with stirring. During this addition, the pH dropped slightly below 7 as glutaraldehyde liberates H$^+$ when in contact with H$_2$O. The pH was adjusted to 8.2 with a 10% solution of sodium hydroxide to obviate any acid denaturation of the enzyme, followed by one hour of agitation at a temperature of 15 to 18°C, during which a water-insoluble polymer formed in which the cells were entrapped and immobilized in the finished polymer matrix.

The insoluble material was separated by centrifugation, extruded with conventional equipment, known in the art and similar to extruder model EXDS-60 (supplied by Fugi Pandal Co., Ltd., Kyoto Osaka, Japan), and allowed to air dry at room temperature until the moisture content was less than 20% which was a period of 2-3 days. The dried composite matrix (32 grams) was ground to a fairly uniform particle size (40-60 pounds/foot$^3$ bulk density, 0.64 to 0.92 g/ml), which was determined using a weight measurement of 100 ml of volume of immobilized material. The composition of the composite matrix is given in the Table below.

TABLE IA

COMPOSITION OF FINISHED IMMOBILIZED CELL CATALYST BASED ON FEED REACTANT

|  | Reaction Mixture Actual Grams | Percent by Wt. | Andydr. Grams | Resultant Matrix Composition Estimate % by Wt. |
| --- | --- | --- | --- | --- |
| Polyethylene-imine (*) | 83 | 12.7 | 4.1 | 15.4 |
| Chitosan (**) | 235 | 36.0 | 1.1 | 4.1 |
| Glutaralde-hyde (***) | 15 | 0.3 | 1.5 | 5.6 |
| Whole Cells | 330 | 50.5 | 20.0 | 74.9 |
| TOTAL | 663 | 100.0 | 26.7 | 100.0 |

(*)     ml of 5% w/v aqueous solution assumed equivalent to grams

(**)     ml of 0.5% w/v dilute aqueous acetic acid solution assumed equivalent to grams

(***)     ml of 10% w/v aqueous solution assumed equivalent to grams

BIOORGANIC TRANSFORMATION OF CINNAMIC ACID TO L-PHENYLALANINE

The immobilized cell matrix was combined with cinnamic acid substrate solutions containing ammonium chloride prepared as described above to determine effectiveness in producing L-phenylalanine from trans cinnamic acid, using a column procedure as follows.

COLUMN PROCEDURE

25 grams of the dry immobilized cell matrix (extruded, air dried and ground) were rehydrated overnight for about 16 hours in 100 ml of the 0.5% cinnamic acid substrate solution (0.0337 M) prior to being packed into a glass column (1 cm internal diameter and 105 cm high). The rehydration swells the immobilized dry form whereby the enzyme is activated. The cell volume was 40 cm$^3$. 1% cinnamic acid substrate solution - (0.0675 M) was fed up-flow under continuous operation (pH 9.3), at a constant flow rate of 4 ml/hour, constant take-off rate (removal of reacted substrate), and constant temperature (in/out 25/25°C). The bioorganic conversion to L-Phenylalanine in the eluate was determined as follows.

CHARACTERIZATION OF L-PHENYLALANINE

300 ml of the eluate was adjusted to a pH of 1.5 to 2.0 with hydrochloric acid, causing 1.05 grams of unconverted trans cinnamic acid to precipitate which was separated by filtration. The dilution is too great for L-phenylalanine to precipitate, whereas trans cinnamic acid is essentially insoluble under these conditions. Thus, next the filtrate was washed with ether and the aqueous layer concentrated to about 1/5th of its volume under vacuum. 2.2 grams of L-Phenylalanine were then precipitated by adjusting the pH to 5.5 with 10% NaOH aqueous solution, and isolated by filtration (crude yield 67.7% L-phenylalanine). The crude crystals were dissolved in a very small volume of 1.5 N NaOH and reprecipitated by addition of 50% acetic acid. The pure L-Phenylalanine was separated by filtration and dried for 12 to 24 hours in an oven at 100°C. 1.9 grams of the pure crystals were isolated, which is a yield of 64.4%. A qualitative estimation of the formed L-Phenylalanine in the medium was carried out by thin-layer chromatography (TLC) using the ascending technique with silica gel plates (Eastman Chromatogram sheets #13179). Developing solvent: 1-butanol:acetic acid:water in an amount of 4:1:1 pts./vol. Plate size was about 2 1/4 $\times$ 2 1/4 inches (about 5.7 $\times$ 5.7 centimeters). Developing time was about one hour. The plates were air dried in an oven at 50°C for 5-10 minutes to remove the solvent and then sprayed with ninhydrin solution and developed by heating at 65-75°C for 15 minutes. The colored spots were visually compared with references of known quantities of authentic L-Phenylalanine.

The results are summarized in the Tables below. The process design parameters are given in Table IB, and catalyst productivity information over time in Table IC.

## TABLE IB

### LABORATORY PROCESS DESIGN PARAMETERS FOR IMMOBILIZED CELL L-PHENYLALANINE FIXED BED COLUMN VIA ENZYMATIC CATALYSIS

| | |
|---|---|
| dry ground starting material, grams | 25 |
| reactor type | adiabatic feed bed |
| reactor dimension (diameter x height), cm | 1 x 105 |
| catalyst volume per reactor, cm³ | 40 (25 g dry) |
| dry catalyst bulk density, lb/ft³ (g/ml) | 40-60 (0.64-0.92) |
| average catalyst productivity, g of L-phe/1 hour | 6.5 (first 39 days) |
| operating temperature (in/out), °C | 25/25 |
| aqueous feed substrate cinnamic acid, mM | 67.5 |
| $NH_3$ (mM) | 3482 |
| feed $NH_3$/cinnamic acid ratio (molar) | 51/1 |
| aqueous feed substrate pH | 9.3 |
| residence time, hours average | 10 |
| cinnamic acid conversion, %/pass | 65 (±3) |
| effluent L-phe concentration, g/liter | 5.8 |
| projected immobile enzyme catalyst half life, days | 80-100 (50% of original activity) |

## TABLE IC

### BIOTRANSFORMATION DATA SUMMARY

| | |
|---|---|
| Column Size: | 1.0 x 105 cm |
| Column Capacity: | 82 cm³ |
| Dry Catalyst: | 25 grams |
| Feed Substrate Concentration: | 67.5 mM (1% w/v) |
| Ratio $NH_3$/Cinnamic Acid: | 51/1 |
| pH: | 9.2 |
| Flow Rate, ml/hr: | 4 |
| Residence time, hours: | 10 |

## TABLE IC (Continued)

| Running Time Days | % of Original Activity | Column Effluent Conc. of L-phe (% by vol.) | Total L-phe Produced (grams) | Cell Productivity (grams of L-phe/ grams of Immob. Cells) |
|---|---|---|---|---|
| 3 | 100 | 0.60 | 2.03 | |
| 4 | 100 | 0.60 | 2.86 | |
| 5 | 100 | 0.50 | 3.14 | |
| 6 | 100 | 0.60 | 3.78 | |
| 7 | 100 | 0.60 | 4.19 | |
| 10 | 100 | 0.60 | 5.92 | 0.237 |
| 11 | 100 | 0.60 | 6.50 | 0.26 |
| 12 | 100 | 0.60 | 7.08 | 0.28 |
| 13 | 100 | 0.60 | 7.66 | 0.306 |
| 14 | 100 | 0.60 | 8.24 | 0.33 |
| 17 | 100 | 0.60 | 9.96 | 0.398 |
| 21 | 100 | 0.60 | 12.26 | 0.49 |
| 26 | 97 | 0.60 | 15.14 | 0.606 |
| 27 | 96 | 0.60 | 15.72 | 0.63 |
| 30 | 100 | 0.65 | 18.02 | 0.721 |
| 32 | 97 | 0.63 | 18.62 | 0.745 |
| 33 | 94 | 0.61 | 19.21 | 0.768 |
| 34 | 94 | 0.61 | 19.80 | 0.792 |
| 35 | 92 | 0.60 | 20.38 | 0.815 |
| 38 | 92 | 0.60 | 22.11 | 0.884 |
| 39 | 92 | 0.60 | 22.69 | 0.908 |
| 40 | 91 | 0.59 | 23.26 | 0.930 |
| 41 | 91 | 0.59 | 23.83 | 0.953 |
| 42 | 91 | 0.59 | 24.40 | 0.976 |
| 45 | 88 | 0.57 | 26.04 | 1.042 |
| 46 | 86 | 0.56 | 26.57 | 1.063 |
| 48 | 88 | 0.57 | 27.65 | 1.106 |
| 49 | 88 | 0.57 | 28.20 | 1.128 |
| 55 | 85 | 0.55 | 31.44 | 1.258 |
| 61 | 82 | 0.53 | 34.45 | 1.378 |
| 66 | 77 | 0.50 | 36.88 | 1.475 |
| 70 | 75 | 0.49 | 38.68 | 1.547 |
| 75 | 69 | 0.47 | 40.96 | 1.638 |
| 77 | 69 | 0.47 | 41.53 | 1.661 |

EXAMPLE II

CELL ISOLATION

A cell suspension from another 100 liter fermentation as described above was centrifuged, followed by decantation of the remaining liquid to remove the whole cells from the broth. Yield: 1630 grams (2.0% wt. vol.).

IMMOBILIZATION PROCEDURE

An 800 gram aliquot from the above cell centrifugate was treated immediately after isolation (no cold storage) as follows. It was diluted under agitation with 1500 ml of deionized water and the resulting suspension cooled at 15°C externally. During all the steps the temperature was maintained between 10-15°C. To the suspension was added with stirring 300 ml of 5% w/v aqueous solution of polyethyleneimine (average molecular weight of 60,000) over a period of 10 minutes and agitation continued for 30 minutes. Then, 810 ml of a 0.5% w/v aqueous dilute acetic acid solution of chitosan was added over 2 minutes and agitation continued for 15 minutes. Next, 54 ml of a 10% w/v aqueous solution of glutaraldehyde was added with stirring over a time period of 5 minutes. During this addition, the pH dropped to about 7.5 and was readjusted to 8.2 with about 6.5 ml of 10% sodium hydroxide solution. This was followed by a 60 minute period of agitation, during which a water-insoluble polymer formed, in which the cells were entrapped and immobilized in the finished polymer matrix.

The insoluble material was separated by centrifugation, extruded with conventional equipment, as in Example I above, and allowed to air dry for 3 days at room temperature. (There was no grinding.) Dry weight was 45 grams of immobilized cell matrix. The composition of the matrix is given in the Table below.

## TABLE IIA

### COMPOSITION OF FINISHED IMMOBILIZED
### CELL CATALYST BASED ON FEED REACTANT

|  | Reaction Mixture Actual Grams | Percent by Wt. | Anhydr. Grams | Resultant Matrix Composition Estimate % by Wt. |
|---|---|---|---|---|
| Polyethylene-imine (*) | 300 | 15.3 | 15.0 | 32.3 |
| Chitosan (**) | 810 | 41.3 | 4.0 | 8.6 |
| Glutaralde-hyde (***) | 54 | 2.7 | 5.4 | 11.6 |
| Whole cells | 800 | 40.7 | 22.0 | 47.5 |
| TOTAL | 1964 | 100.0 | 46.4 | 100.0 |

(*)    ml of 5% w/v aqueous solution assumed
       equivalent to grams

(**)   ml of 0.5 w/v aqueous solution assumed
       equivalent to grams

(***)  ml of 10% w/v aqueous solution assumed
       equivalent to grams

## BIOORGANIC TRANSFORMATION OF CINNAMATE TO L-PHENYLALANINE

The immobilized cell matrix was combined with cinnamic acid substrate solutions containing ammonium chloride prepared as described above to determine effectiveness in producing L-phenylalanine from trans cinnamic acid, using a column procedure as follows.

## COLUMN PROCEDURE

37 grams of the immobilized cell matrix (extruded and air dried) were rewetted for 16 hours in 100 ml of 0.5% cinnamic acid solution (0.0337 M) and then packed into a column (1.5 cm internal diameter and 100 cm height). The cell volume was 80 cm$^3$. 1% cinnamic acid substrate solution (0.0675 M) was fed up-flow under continuous operation (pH 9.3), at constant flow rate of 10 ml/hour, constant take-off rate (removal of reacted substrate), and constant temperature (in/out 25/25°C). The bioorganic conversion to L-phenylalanine in the eluate was determined spectrophotometrically as follows and found to be about 54%.

## SPECTROPHOTOMETRIC ASSAY TO CHARACTERIZE L-PHENYLALANINE

The L-phenylalanine content of the eluate solution was determined spectrophotometrically by measuring the amount of unconverted trans-cinnamic acid (starting material) remaining in the eluate solution. The L-phenylalanine content can then be calculated by the following equation:

$$A - B \times \frac{165}{148} = \% \text{ L-phe}$$

where A is the original trans-cinnamic acid content of the starting material in percent, B is the remaining, uncovered trans-cinnamic acid content of the eluate solution in percent, 165 is the molecular weight of L-phe, and 148 is the molecular weight of trans-cinnamic acid.

## PROCEDURE

Preparation of Reagents:

A. 1% Trans-Cinnamic Acid Solution

    1. 20 ml concentrated NH$_4$OH (28.6% NH$_3$)
    2. 50 ml deionized H$_2$O
    3. 1.00 gram trans-cinnamic acid
    4. 15 ml concentrated HCl (38%), adjust pH to 9.2-9.3
    5. Add deionized H$_2$O to 100 ml

B. 1.115% L-Phe Solution

    1. 20 ml concentrated NH$_4$OH
    2. 50 ml deionized H$_2$O
    3. 1.115 gram L-phe
    4. 15 ml concentrated HCl (38%), adjust pH to 9.2-9.3
    5. Add deionized H$_2$O to 100 ml

C. Diluent Solution

    1. 20 ml concentrated NH$_4$OH (28.6% NH$_3$)
    2. 50 ml deionized H$_2$O
    3. 15 ml concentrated HCl (38%), adjust pH to 9.2-9.3

4. Add deionized H$_2$O to 100 ml

## STANDARD CURVE PREPARATION

To prepare a standard curve, 5 control solutions were prepared from the above reagents as follows.

| (Combined A+B) % Cinnamic Acid Control | ml of Reagent A | ml of Reagent B |
|---|---|---|
| 0.1 | 0.5 | 4.5 |
| 0.3 | 1.5 | 3.5 |
| 0.5 | 2.5 | 2.5 |
| 0.7 | 3.5 | 1.5 |
| 0.9 | 4.5 | 0.5 |

Reagents A and B were combined and mixed well, and 150 microliters (0.15 ml) of the mixture were pipetted to a 100 ml volumetric flask and diluted to mark with distilled water. After thorough mixing, absorbance was read on the spectrophotometer at 290 nm wavelength. A standard curve was then plotted from the absorbance values obtained, and the activity over 1 to 62 days compared thereto (see Table IIC).

## ASSAYS OF SAMPLES

The eluate solution was assayed as is, since the starting material trans-cinnamic acid level was 1.0%. - [Higher levels of trans-cinnamic acid (i.e. Example III below) must first be diluted to the equivalent of 1.0% using diluent "C".] To assay, 150 microliters (0.15 ml) of eluate solution was added to a 100 ml volumetric flask and diluted to mark with distilled water. After mixing well, absorbance was read on the spectophotometer at 290 nm wavelength. The trans-cinnamic acid content of the sample was found by using the standard curve prepared previously and from this the level of L-phe was calculated using the equation above. The sample was also qualitatively assayed by TLC to check for impurities and identify the products, by the same procedure as in Example I above.

The results are summarized in the Tables below. The process design parameters are given in Table IIB, and catalyst productivity information over time in Table IIC.

## TABLE IIb

### LABORATORY PROCESS DESIGN PARAMETERS
### FOR IMMOBILIZED CELL L-PHENYLALANINE FIXED
### BED COLUMN VIA ENZYMATIC CATALYSIS

| | |
|---|---|
| reactor type | adiabatic fixed bed |
| reactor dimention (diameter x height) cm | 1.5 x 100 |
| catalyst volume, cm³ | 80 (37 g dry) |
| dry catalyst bulk density, lb/ft³ (g/cm³) | 40-60 (0.64-0.92) |
| average catalyst productivity, g of L-phe/1 hour | 6.0 (first 16 days) |
| operating temperature (in/out), °C | 25/25 |
| aqueous feed substrate composition cinnamic acid, mM | 67.5 |
| $NH_3$ (mM) | 3482 |
| feed $NH_3$/cinnamic acid ratio (molar) | 51/1 |
| aqueous feed substrate pH | 9.3 |
| residence time, hours average | 8 |
| cinnamic acid conversion, %/pass | 54 |
| projected immobilized enzyme catalyst half life, days | 60-80 (50% of original activity) |
| effluent L-phe concentration g/l | ranges 6-5 |

## TABLE IIC

### BIOTRANSFORMATION DATA SUMMARY

| | |
|---|---|
| Column Size: | 1.5 x 100 cm |
| Column Capacity: | 180 ml |
| Dry Catalyst: | 37 grams |
| Feed Substrate Concentration: | 67.5 mM (1% w/v) |
| Ratio $NH_3$/Cinnamic Acid: | 51/1 |
| pH: | 9.3 |
| Flow Rate, ml/hour: | 10 |
| Residence time, hours: | 8 |

## TABLE IIC (Continued)

| Running Time Days | % of Original Activity | Column Effluent Conc. of L-phe (% by vol.) | Total L-phe Produced, Grams | Cell Productivity (grams of L-phe/grams of Immob. Cells) |
|---|---|---|---|---|
| 1 | 100 | 0.70 | 2.34 | |
| 2 | 100 | 0.60 | 3.78 | |
| 5 | 100 | 0.60 | 8.10 | |
| 6 | 100 | 0.60 | 9.54 | 0.258 |
| 7 | 100 | 0.60 | 10.98 | 0.297 |
| 8 | 100 | 0.60 | 12.42 | 0.336 |
| 9 | 100 | 0.60 | 13.86 | 0.375 |
| 12 | 100 | 0.60 | 18.18 | 0.491 |
| 16 | 100 | 0.60 | 23.94 | 0.642 |
| 20 | 97 | 0.58 | 30.90 | 0.835 |
| 21 | 93 | 0.56 | 32.24 | 0.871 |
| 25 | 90 | 0.54 | 37.42 | 1.011 |
| 26 | 90 | 0.54 | 38.72 | 1.046 |
| 27 | 87 | 0.52 | 39.97 | 1.080 |
| 28 | 87 | 0.52 | 41.22 | 1.114 |
| 29 | 85 | 0.51 | 42.44 | 1.147 |
| 33 | 87 | 0.52 | 46.18 | 1.248 |
| 34 | 87 | 0.52 | 47.43 | 1.282 |
| 35 | 83 | 0.50 | 48.63 | 1.314 |
| 36 | 82 | 0.49 | 49.81 | 1.346 |
| 37 | 80 | 0.48 | 50.96 | 1.377 |
| 40 | 78 | 0.47 | 54.34 | 1.469 |
| 41 | 78 | 0.47 | 55.47 | 1.499 |
| 42 | 77 | 0.46 | 56.57 | 1.529 |
| 43 | 77 | 0.46 | 57.67 | 1.559 |
| 44 | 77 | 0.46 | 58.77 | 1.588 |
| 47 | 70 | 0.42 | 61.79 | 1.670 |
| 50 | 68 | 0.41 | 63.75 | 1.723 |
| 54 | 59 | 0.39 | 68.47 | 1.851 |
| 57 | 62 | 0.37 | 70.88 | 1.916 |
| 62 | 53 | 0.32 | 74.00 | 2.000 |

## EXAMPLE III

## IMMOBILIZATION PROCEDURE

Another 800 g aliquot of cells from the whole broth from Example II was immobilized, and the resultant extruded and air dried in the same manner to provide 45 g of immobilized matrix.

## COLUMN PROCEDURE

The 45 grams of the immobilized cell matrix were rewetted in 0.5% w/v cinnamic acid substrate (0.0337 M) and then packed into a column (2.6 cm internal diameter and 40 cm high). 3% w/v cinnamic acid substrate solution (0.202 M) prepared as described above was fed up-flow under continuous operation (pH 9.2), at constant flow rate (3.5 ml/hour), constant take-off rate (removal of reacted substrate), and a near constant in/out temperature of 25/25°C±2. The rate of conversion of trans cinnamic acid to L-phe in the eluate was determined spectrophotometrically, in the manner described in Example II above, except the solution was diluted to 1% with diluent "C" described above since the starting substrate here was 3%. Conversion was found to be about 49%. The half life of 33 days is less than that of Example I, as the greater concentration of 3% substrate produced L-phe faster than the lesser concentration of 1% substrate in Example I. Process design parameters are given in Table IIIA and catalyst productivity over time is in Table IIIB. A qualitative assay was also conducted as in Example I by TLC for product identification and purity.

## TABLE IIIA

## LABORATORY PROCESS DESIGN PARAMETERS FOR
## IMMOBILIZED CELL L-PHENYLALANINE FIXED BED
## COLUMN VIA ENZYMATIC CATALYSIS

| | |
|---|---|
| reactor type | adiabatic fixed bed |
| reactor dimension (diameter x height), cm | 2.6 x 40 |
| catalyst volume per reactor, cm³ | 100 (45 g dry) |
| dry catalyst bulk density, lb./ft.³ (g/cm³) | 40-60 (0.64-0.92) |
| average catalyst productivity, g of L-phe/1 hr. | 14-16.5 |
| operating temperature (in/out), °C | 25/25 (±2°C) |
| aqueous feed substrate composition 3% cinnamic acid, mM | 202 |
| $NH_3$, mM | 3482 |
| feed $NH_3$/cinnamic acid ratio (molar) | 17/1 |
| aqueous feed substrate pH | 9.2 |
| residence time, hours average | 26 |
| cinnamic acid conversion, %/pass | highest 49.3 |
| effluent L-phe concentration, g/1 | ranges 12-16 |
| effluent cinnamic acid concentration, g/1 | ranges 18-14 |
| half life of catalyst per pass, optimal | 33 days (projected) |

## TABLE IIIB

COLUMN SIZE 2.6 x 40 cm          3.0% w/v FEED

| Running Time - Days | % of Original Activity |
|---------------------|------------------------|
| 7 | 100 |
| 11 | 97 |
| 16 | 93 |
| 17 | 87 |
| 18 | 85 |

EXAMPLE IV

FREE CELL DATA

A cell suspension from an entire fermentation broth prepared as described above was centrifuged followed by decantation of the remaining liquid to separate the centrifugate containing the whole cells. The cells were washed with an equal amount by volume of 0.9% saline solution by resuspending and centrifuging again, and then 10 grams of free whole *S. roseus* cells containing PAL were shaken on a gyratory shaker at 20°C in 30 ml of 1% cinnamic acid substrate (0.0675 M) at pH 10.0.

After 20 hours the cells were removed by centrifugation and the substrate assayed by TLC. The conversion of trans-cinnamic acid to L-phenylalanine was estimated to be approximately 54%. The cells were reused again by shaking in 30 ml of fresh 1% w/v cinnamic acid substrate for 20 hours under the same conditions. After removing the cells by centrifugation the substrate was assayed again by TLC. The conversion was only 8%.

The third use of the cells resulted in no reaction after 20 hours. Thus, it can be seen that while this batch process gives an initially good bioconversion of 54%, it does not readily lend itself to reuse as the immobilized systems described above in Examples I, II, and III.

EXAMPLE V

The fermentation conditions were varied from that used in the Examples above to see the effect on yield of wet cells. The results are summarized in the Table below.

## TABLE V

### EFFECT OF CULTURE MEDIUM, TIME AND TEMPERATURE
### OF *S. roseus* (ATCC ‡28988) ON WET CELL YIELD

Cultivation Temperature Range: 18-27°C
Cultivation Time Range: 41-68 Hours
Cultivation Unit: 1000 ml medium
Fernbach flask

| Sample Reference Number | Ingredients, % w/v | | | | time (hours) /temp°C | Yield*** g/1000 ml | Note |
|---|---|---|---|---|---|---|---|
| | Peptone* | Yeast Extr.** | NaCl | L-Phe | | | |
| 151a | 0.5 | 2.0 | 0.5 | --- | 64/18 | 33 | |
| 151e | 0.5 | 2.5 | 0.5 | --- | 64/18 | 38 | |
| 151d | 0.5 | 3.0 | 0.5 | --- | 64/18 | 44 | |
| 151f | 0.5 | 2.5 | 0.5 | --- | 64/18 | 40 | |
| 149 | 0.5 | 2.0 | 0.5 | 0.05 | 64/18-20 | 30 | (1) |
| 145 | 0.5 | 2.0 | 0.5 | 0.05 | 64/18 | 33 | |
| 143 | 0.5 | 2.0 | 0.5 | 0.05 | 64/20 | 30 | |
| 138A | 0.5 | 1.0 | 0.5 | 0.05 | 68/19-20 | 22 | |
| 138B | 1.0 | 1.0 | 0.5 | 0.05 | 68/19-20 | 26 | |
| 138c | 0.5 | 2.0 | 0.5 | 0.05 | 68/19-20 | 40 | |
| 132 | 0.5 | 1.5 | 0.0 | 0.05 | 65/25 | 22 | |
| 131 | 0.5 | 1.5 | 0.5 | 0.05 | 67/20-21 | 25 | |
| 129 | 0.5 | 1.0 | 0.5 | 0.05 | 48/24-25 | 17 | |
| 122 | 0.5 | 1.0 | 0.5 | 0.05 | 48/25 | 20 | |
| 117 | 0.5 | 1.0 | 0.5 | 0.05 | 67/27 | 8 | |
| 116 | 0.5 | 1.0 | 0.5 | 0.05 | 48/20-22 | 20 | |
| 115 | 0.5 | 1.0 | 0.5 | 0.05 | 66/30 | no cells | |
| 112 | 0.5 | 1.0 | 0.5 | 0.05 | 66/25 | 20 | |
| 105 | 0.5 | 1.0 | 0.5 | 0.1 | 46/25 | 23 | |
| 102A | 0.5 | 1.0 | 0.5 | 0.1 | 41/25 | 20 | (1) |
| 102B | 0.5 | 1.0 | 0.5 | 0.1 | 44/25 | 25 | (1) |
| 100 | 0.5 | 1.0 | 0.5 | 0.05 | 64/20 | 18 | (2) |
| 94 | 0.5 | 1.0 | 0.5 | 0.05 | 44/25 | 21 | (3) |

20

Notes: (1)   800 ml in Fernbach flask
       (2)   666 ml in Fernbach flask
       (3)   600 ml in Fernbach flask

\*       Bacto-Peptone, Difco Certified, 0118-01-8,
         Difco Labs., Detroit

\*\*      Yeast Extract, Difco Certified, 0127-01,
         Difco Labs., Detroit

\*\*\*     Cell growth is based on cultivation time of
         72 hours at 20°C (±) 1 in 2800 ml Fernbach
         flasks on shakers at 250 RPM, centrifuged
         and once washed with 0.9% NaCl
         solution and centrifuged again.

The cultivation procedure was 1000 ml of medium in a 2800 ml Fernbach flask unless otherwise noted. Sterilization was for 45 minutes at 121°C. The flasks were inoculated with 10 ml of seed culture prepared as previously described, and then incubated on a gyratory shaker at 250 rpm for the specified temperature and time. Cells were harvested by centrifugation from the whole culture broth and washed with an equal volume of 0.9% saline solution by resuspending and centrifuging again.

It was found that cell growth during cultivation depends not only on the composition of the culture media, but also the cultivation temperature and time. Cell growth was observed between 41-68 hours at temperatures ranging from 18-27°C. At 30°C, no cell growth was observed (Sample No..115).

In general, the aqueous fermentation medium comprises about 0.5 to 1% w/v peptone, about 1 to 3% w/v yeast extract, about 0 to 1% w/v NaCl, and about 0 to 0.1% w/v L-phe, and the temperature is maintained under about 27°C. The optimal conditions appear to be 0.5% w/v peptone, 2 to 3% w/v yeast extract, 0.5% w/v NaCl, and 0.05% w/v L-phe, at a temperature of 18 to 25°C. In particular, comparing sample Nos. 151a, 151f, and 138c, it can be seen that a little L-phe in the fermentation media may obtain acceptable results with the use of less yeast extract.

## Claims

1. A method for the immobilization of a phenylalanine ammonia-lyase producing *Sporobolomyces roseus* which method comprises forming a reaction product by the steps of:

a) at a temperature less than about 27°C, introducing a solution of a polyamine having pendant amino groups to an aqueous medium containing a mass of cells of *S. roseus*;

b) adding a multifunctional amine reactive material at a pH above about 7.0 and an aqueous solution of chitosan to the aqueous medium to form a cross-linked reaction product; and

c) removing the cross-linked reaction product from the aqueous medium to provide an immobilized cell mass end product capable of catalyzing the biotransformation of trans cinnamic acid to L-phenylalanine.

2. The method of Claim 1 wherein the polyamine is polyethylenediamine, polyethylenimine, polydiethylenetriamine, polytriethylenetetramine, polypentaethylene-hexamine, polyhexamethylenediamine, poly-hexamethylenediamine, polymethylenedicyclohexylamine, polymethylenedianiline, polytetraethylenepen-tamine, polyphenylenediamine, or a copolymer of epihalohydrin and a polyamine.

3. The method of Claims 1 or 2 wherein the polyamine is polyethyleneimine (PEI) having a molecular weight of at least about 500 Daltons with a primary amino group content of at least 10 weight percent of the PEI's amino groups.

4. The method of any of the Claims 1 to 3 wherein the PEI is placed in aqueous solution at a concentration of about 1 to 10 weight percent before its introduction to the aqueous medium.

5. The method of any of the Claims 1 to 3 wherein the amount of PEI is from about 2 to 22 weight percent of the immobilized cell mass.

6. The method of any of the Claims 1 to 5 wherein the polyamine has a molecular weight range of from about 500 to 100,000.

7. The method of any of the Claims 1 to 6 wherein the multifunctional amine reactive material is a multifunctional aldehyde, a multifunctional organic halide, a multifunctional anhydride, a multifunctional azo compound, a multifunctional isothiocyanate or a multifunctional isocyanate.

8. The method of Claim 7 wherein the multifunctional amine reactive material is bis-diazobenzidine-2,2'-disulfonic acid; 4,4'-difluoro-3,3'-dinitrodiphenylsulfone; diphenyl-4,4'-dithiocyanate-2,2'-disulfonic acid; 3-methoxydiphenylmethane-4,4'-diisocyanate; toluene-2-isocyanate-4-isothiocyanate; toluene-2,4-diisothiocyanate; diazobenzidine; diazobenzidine-3,3'-dianisidine; N,N'-hexamethylene bisiodoacetamide; hexamethylene diisocyanate; cyanuric chloride; 1,5-difluoro-2,4-dinitrobenzene; or glutaraldehyde.

9. The method of any of the Claims 1 to 8 wherein the multifunctional amine reactive material is glutaraldehyde employed in an amount of from about 4 to 26 weight percent of the immobilized cell mass preparation.

10. The method of any of the Claims 1 to 9 wherein the amount of chitosan employed is from about 0.5 to 22 weight percent of the immobilized cell mass preparation.

| | DOCUMENTS CONSIDERED TO BE RELEVANT | | EP 86117965.3 |
|---|---|---|---|
| Category | Citation of document with indication, where appropriate, of relevant passages | Relevant to claim | CLASSIFICATION OF THE APPLICATION (Int. Cl.4) |
| D,A | CHEMICAL ABSTRACTS, vol. 95, no. 11, September 14, 1981, (Columbus, Ohio, USA)<br><br>TANABE SEIYAKU CO.,LTD "L-Phenyl-alanine"<br>page 463, column 2, abstract no. 95 470b<br><br>& Japan. Kokai 81 26,197<br><br>-- | 1 | C 12 N 11/10<br>C 12 N 11/02<br>C 12 N 9/88 |
| D,A | CHEMICAL ABSTRACTS, vol. 89, no. 25, December 18, 1978 (Columbus, Ohio,USA)<br><br>I.CHIBATA et al. "Enzymatic pro-duction of L-phenylalanine"<br>page 452, column 1, abstract no. 213 580p<br><br>& Japan. Kokai 78 96,388 (TANABE SEIYAKU CO.,LTD)<br><br>-- | 1 | |
| A | US - A - 4 089 746 (MASRI et al.)<br>* Claims 1-6 *<br><br>-- | 1 | TECHNICAL FIELDS SEARCHED (Int. Cl.4)<br><br>C 12 N<br>C 12 P |
| D,A | GB - A - 2 092 161 (SEARLE)<br><br>---- | | |

The present search report has been drawn up for all claims

| Place of search | Date of completion of the search | Examiner |
|---|---|---|
| VIENNA | 02-04-1987 | BECKER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82